# EUROPÄISCHE PATENTANMELDUNG

(11) **EP 0 999 276 A1**
(43) Veröffentlichungstag der Anmeldung: **10.05.2000**
(21) Anmeldenummer: 99120314.2
(22) Anmeldetag: 12.10.1999
(51) Int. Cl.: C12N 15/67, C12P 21/02, C07K 14/21, C12N 9/20, C12N 15/31, C12N 15/55

(54) **Verfahren zur Herstellung extrazellulärer Proteine durch Expression in heterologen Wirtsbakterien**

(30) Priorität: 17.10.1998 DE 19848016
(71) Anmelder: Roche Diagnostics GmbH, 68298 Mannheim (DE)
(72) Erfinder: Jaeger, Karl Erich, Dr., 45479 Muehlheim an der Ruhr (DE); Schmuck, Rainer, Dr., 83671 Benediktbeuern (DE); Schneidinger, Bernd, Dr., 83670 Bad Heilbrunn (DE); Rosenau, Frank, 45549 Sprockhoevel (DE)

(57) **Zusammenfassung**

Verfahren zur Herstellung eines extrazellulären, mikrobiellen Proteins durch heterologe Expression, wobei neben den für das bzw. die gewünschten Proteine kodierenden Nukleinsäuremoleküle ein für einen Sekretionsapparat kodierendes Nukleinsäuremolekül in ein Gram-negatives Bakterium transformiert sind. Besonders geeignet hat sich das Verfahren für die Expression und Sekretion extrazellulärer Proteine mit esterolytischer Aktivität, wie beispielsweise Lipase oder Elastase, in Stämmen der Gattung *Pseudomonas* erwiesen.

## Beschreibung

Gegenstand der Erfindung ist ein Verfahren zur Herstellung eines extrazellulären, mikrobiellen Proteins, wie beispielsweise Lipase, durch heterologe Expression, wobei neben dem für das gewunschte Protein kodierenden Nukleinsäuremolekül ein für einen Sekretionsapparat kodierendes Nukleinsäuremolekül in ein Gram-negatives Bakterium inseriert ist.

Die Expression heterologer Gene in Bakterien stellt gegenwärtig die einfachste und wirtschaftlichste Methode dar, kommerziell interessante Proteine in großer Menge zu gewinnen. Zahlreiche biotechnologisch interessante Proteine werden jedoch aus Bakterien gewonnen, die nach dem Gentechnikgesetz (§7, GenTSV) zur Sicherheits- oder Risikostufe 2 (S2) gehören und deren Anzucht im technischen Maßstab daher nicht nur ein aufwendiges Genehmigungsverfahren erfordert, sondern auch hohe Kosten für eine Spezialausstattung der Produktionsstätten bedingt. Werden hingegen definierte Gene aus "S2-Bakterien" in heterologen "nicht-S2-Bakterien" exprimiert, so kann bei entsprechenden Behörden die Eingruppierung der entstandenen gentechnisch veränderten Organismen (GVO's) in die Sicherheitsstufe S1 beantragt werden, was erhebliche Erleichterungen in Laboratorien und Produktionsbereichen bedingt.

Das zur Sicherheitsstufe 2 gehörende Bakterium der Spezies *Pseudomonas aeruginosa* ist beispielsweise bekannt als ein Mikroorganismus, der zahlreiche Proteine, insbesondere Enzyme mit biotechnologischer Bedeutung, wie beispielsweise Lipase, bildet, die über den "General Secretory Pathway" (A.P. Pugsley, Microbiol. Rev. 57, (1997) 50-108) exportiert werden. Der Transport über die Cytoplasmamembran in das Periplasma erfolgt dabei mit Hilfe eines unspezifischen Transportapparats (des sg. *sec*-Systems), der vermutlich in allen Bakterien vorkommt (M. Pohlschröder et al., Cell 28 (1997) 563-566. Das zu sekretierende Protein wird in der Regel beim oder nach dem Durchtritt durch die innere Cytoplasmamembran gefaltet, wobei Isomerasen, wie Prolyl-Peptidyl-*cis/trans*-Isomerasen, Disulfid-Oxidoreduktasen sowie -Isomerasen und spezifische Chaperone als Katalysatoren dienen (S. Raina und D. Missiakis, Annu. Rev. Microbiol. 51 (1997) 179-202). Im Periplasma befinden sich enzymatisch aktive Enzyme, die in einem zweiten Schritt mittels eines spezifischen Sekretionsapparats durch die äußere Membran transportiert werden. Der für *Pseudomonas aeruginosa* spezifische Sekretionsapparat besteht beispielsweise aus zwölf verschiedenen Proteinen, deren Lokalisation nur in Teilen bekannt ist (A. Lazdunski et al., in: Molecular Biology of *Pseudomonas*; eds.: T. Nakazawa et al., ASM -Press Washington (1996), 427-437). Dem Sekretionsapparat von *P*. *aeruginosa* analoge Sekretionssysteme finden sich in zahlreichen Gram-negativen Bakterien, in der Regel sind diese Sekretionsssysteme jedoch spezifisch für Proteine aus dem homologen Wirt. Die Sekretion von Proteinen aus anderen Mikroorganismen, die beispielsweise der Sicherheitsstufe 2 oder höher zugeordnet sind, kann nur erfolgen, wenn ein für diese Mikroorganismen spezifischer Sekretionsapparat vorhanden ist. So führte für das bezüglich seiner breiten Anwendung interessante extrazelluläre Enzym Lipase, beispielsweise aus *P*. *aeruginosa*, bis heute lediglich die homologe Expression zu einem entsprechenden Enzym in aktiver Form. Die Synthesrate solcher homologer Expressionssysteme war bisher unter wirtschaftlichen Gesichtspunkten nicht zufriedenstellend (K.-E. Jaeger et al., in: Molecular Biology of *Pseudomonas*; eds.: T. Nakazawa et al., ASM Press Washington (1996). Die heterologe Expression extrazellulärer Proteine, wie etwa die Expression von *P*. *aeruginsoa*-Lipase in *E*.*coli,* führte bisher ebenfalls nicht zu erwünschten Erfolgen, sondern lediglich zu enzymatisch nicht aktiven Formen, die darüber hinaus nicht sekretiert werden.

Der vorliegenden Erfindung liegt somit die Aufgabe zugrunde, ein einfaches und zugleich wirtschaftliches, jedoch hohen Sicherheitsanforderungen genügendes Verfahren zur Herstellung von extrazellulären Proteinen, insbesondere solchen mit esterolytischer Aktivität, zur Verfügung zu stellen.

Die Aufgabe wird gelöst durch ein Verfahren zur Herstellung eines extrazellulären, aus einem Mikroorganismus stammenden Proteins durch heterologe Expression in einem Kulturmedium, dadurch gekennzeichnet, daß man ein für das extrazelluläre Protein kodierendes Nukleinsäuremolekül, ein für einen Sekretionsapparat kodierendes Nukleinsäuremolekül desselben oder eines nahe verwandten Mikroorganismus und gegebenenfalls ein für ein zusätzliches Enzym, welches die korrekte Prozessierung der den Sekretionsapparat bildenden Proteine bewerkstelligt, kodierendes Nukleinsäuremolekül isoliert, in einen Expressionsvektor kloniert und in einem heterologen Gram-negativen Bakterium überexprimiert und anschließend das Protein isoliert. Bevorzugt handelt es sich dabei um extrazelluläre Proteine mit esterolytischer Aktivität bzw, um Esterasen.

Als Quelle für das zu exprimierende extrazelluläre Protein kommen prinzipiell sämtliche Mikroorgansimen der Risikogruppe 2 oder einer höheren Risikogruppe, wie 3 oder 4 in Betracht. Beispielhaft seien hier die Mikroorganismen der Gattung *Pseudomonas* und *Renibacterium* genannt. Folgende Arten der Gattung Pseudomonas sind diesbezüglich erfindungsgemäß bevorzugt: *P*. *aeruginosa*, *P*. *alcaligenes, P*. *anguilliseptica*, *P*. *cepacia*, *P*. *cocovenenans*, *P*. *diminuta*, *P*. *mallei*, *P*. *mendocina* und *P*. *pseudomallei*, wobei extrazelluläre Proteine aus *P*. *aeruginosa* besonders bevorzugt sind.

Das für das jeweils gewünschte Protein kodierende Gen bzw. entsprechende genetische Information tragende Nukleinsäuremoleküle werden aus dem entsprechenden Mikroorganismus isoliert und in (einen) für den Wirtsorganismus passende(n) Expressionsvektor(en) kloniert.

Außerdem wird ein für einen Sekretionsapparat kodierendes Nukleinsäuremolekül in den Expressionsvektor kloniert. Der Sekretionsapparat, wobei es sich in der Regel um einen Multiprotein-Komplex, bevorzugt um ein TypII-Sekretionssystem handelt, ermöglicht den Transport des extrazellulären bzw. mehrerer extrazellulärer Proteine durch die äußere Membran Gram-negativer Bakterien. Das bzw. die für den Sekretionsapparat kodierenden Nukleinsäuremoleküle stammen in der Regel aus demselben oder einem diesem nahe verwandten Mikroorganismus, aus dem das für das jeweilige extrazelluläre Protein kodierende Nukleinsäuremolekül bzw. kodierende Nukleinsäuremoleküle stammen. Beispielhaft seien für einen spezifischen Sekretionsapparat die für einen Multiprotein-Komplex kodierenden *xcp*-Gene aus *P. aeruginosa*-Proteinen angeführt (Tommassen et al., FEMS Microbiol. Rev. 103 (1997), 73-90). Die gesuchten Sekretionsgene werden mittels eines geeigneten Durchmusterungsverfahrens in einer zu erstellenden *P*. *aeruginosa*-Genbank im Cosmid pLAFR3 identifiziert und isoliert. Die xcp-Gene werden - nach Identifizierung einer *P*. *aeruginosa*-Genbank - isoliert und in ein geeignetes Plasmid, wie beispielsweise ein Cosmid (pLAFR3), kloniert. Es können jedoch erfindungsgemäß auch Nukleinsäuremoleküle für den Sekretionsapparat eingesetzt werden, die aus einem unterschiedlichen Organismus als das/die Nukleinsäuremolekül(e) für das/die extrazelluläre(n) Protein(e) stammen. Eine bevorzugte Ausführungsform des erfindungsgemäßen Verfahrens ist dann gegeben, wenn zusätzlich zu den für den Sekretionsapparat kodierenden *xcp*-Genen ein für die Prozessierung der diesen Sekretionsapparat bildenden Proteine zuständiges Gen, wie beispielsweise *xcpA*, exprimiert wird.

Als Wirtsorganismus kommen erfindungsgemäß sämtliche Gram-negativen Bakterien wie *E*.*coli* und insbesondere solche der Gattung *Pseudomonus*, die entweder der Sicherheitsstufe 1 zugeordnet sind oder keiner Sicherheitseinstufüng unterliegen, in Betracht. Beispielhaft seien hier genannt: *P*. *antimicrobica*, *P*. *carboxydohydrogena*, *P*. *fluorescens*, *P*. *mucidolens*, *P*. *putida* und *P*. *woodsii*. Als besonders geeignet haben sich erfindungsgemäß die Stämme *P*. *putida* (z.B. DSM 12384) und *P*. *fluorescens* erwiesen. Eine Probe des Mikroorgansimus DSM 12384 wurde am 20.08.1998 bei der Deutschen Sammlung von Mikroorganismen und Zellkulturen GmbH, Mascheroder Weg 1b, D-38124 Braunschweig hinterlegt.

Wird das für das gewünschte extrazelluläre Protein, wobei es sich vorzugsweise um ein Protein mit esterolytischer Aktivität handelt, zu exprimierende Gen bzw. die für den Sekretionsapparat kodierenden Gene und gegebenenfalls ein für die an der Prozessierung der Sekretionsapparat-Proteine beteiligten Enzyme kodierendes Gen sowie der Wirtsorganismus unter den oben dargelegten Möglichkeiten für das erfindungsgemäße Verfahren entsprechend ausgewählt, wird das überexprimierte Protein in das Kulturmedium sekretiert, woraus es anschließend mittels dem Fachmann bekannter Maßnahmen isoliert werden kann.

Eine bevorzugte Ausführungsform des erfindungsgemäßen Verfahrens ist, wenn der Wirtsorganismus mehrere, beispielsweise zwei, drei, vier oder fünf Nukleinsäuremoleküle, die für dasselbe extrazellulläre Protein oder unterschiedliche extrazelluläre Proteine kodieren, enthält. Die für unterschiedliche Proteine kodierenden Gene werden bevorzugt in separate Expressionsvektoren inseriert und in den Wirtsorganismus transformiert.

Eine besondere Ausführungsform des erfindungsgemäßen Verfahren ist gegeben, wenn das für ein extrazelluläres Protein - insbesondere ist dies der Fall, wenn das Protein esterolytische Aktivität aufweist - kodierende Nukleinsäuremolekül aus zwei oder mehreren funktionellen Einheiten besteht. Die unterschiedlichen Einheiten können entweder in einem Expressionsvektor, oder in zwei separate Expressionsvektoren kloniert und unabhängig voneinander überexprimiert werden. Auch das für den Sekretionsapparat kodierende Nukleinsäuremolekül kann aus einer oder mehreren fünktionellen Einheiten bestehen. Die unterschiedlichen Einheiten können ebenfalls entweder auf einem Expressionsvektor oder verteilt auf mehrere Expressionsvektoren oder in das Genom des Wirtsorganismus integriert vorliegen und unabhängig voneinander überexprimiert werden.

Als vorteilhaft hat sich für das erfindungsgemäße Verfahren erwiesen, wenn das für das extrazelluläre Protein kodierende Nukleinsäuremolekül und/oder das für den Sekretionsapparat kodierende Nukleinsäuremolekül in das Genom des Wirtsorganismus stabil integriert wird. Dies kann beispielsweise durch homologe Rekombination erreicht werden, wie insbesondere unter Verwendung eines sogenannten "suicide"-Vektors. Die Technik der homologen Rekombination ist dem Fachmann bekannt.

Weiterhin hat sich für das erfindungsgemäße Verfahren als vorteilhaft erwiesen, wenn die Expression des für das extrazelluläre Protein kodierenden Gens und/oder das für den Sekretionsapparat kodierende Gen bzw. diese Gene ausmachende Nukleinsäuremoleküle unter der Kontrolle des jeweils natürlichen Promotors oder eines konstitutiven bzw. eines induzierbaren Fremdpromotors stehen.

Besonders vorteilhaft hat sich ferner das erfindungsgemäße Verfahren für aus *P*. *aeruginosa* erhältliche extrazelluläre Proteine mit Lipase- und/oder Elastase- und/oder Protease-Aktivität bzw. deren Expression in dem Wirtsorganismus *P*. *putida* erwiesen. Die funktionellen Einheiten stellen im Falle des für ein extrazelluläres Protein mit Lipase-Aktivität kodierenden Nukleinsäuremoleküls die Gene *lipA* und/oder *lipH* dar, wobei mindestens eines dieser Gene mit einer für eine Signalsequenz eines Proteins kodierenden Nukleinsäureregion versehen ist. Das für extrazelluläre Lipase aus *P*. *aeruginosa* kodierende Operon bestehend aus den Genen *lipA* und *lipH* kann dabei beispielsweise in einen einzigen Expressionsvektor unter Kontrolle des *lac*-Promotors (z.B. in pBBR1MCS) kloniert werden. Das Strukturgen *lipA*, das für extrazelluläre Lipase aus *P*. *aeruginosa* kodiert, und das für die Lipase-spezifische Foldase aus *P*. *aeruginosa* kodierende Gen *lipH* können jedoch darüber hinaus auf unterschiedlichen Expressionsvektoren, unter der Kontrolle induzierbarer und/oder konstitutiver Promotoren stehend, separat von einander kloniert werden. Insbesondere hat sich bei einer solchen Ausführungsform des weiteren als vorteilhaft erwiesen, wenn das Gen *lipA* am 5'-Ende mit der genannten Nukleinsäureregion verbunden ist.

Erfindungsgemäß haben sich insbesondere aus *E.coli* stammende Signalsequenzen, beispielsweise die Signalsequenzen der Proteine OmpA, OmpT oder PhoA, oder aus *Erwinia chrysanthemi* stammende, beispielsweise die des *pelB*-Proteins, als vorteilhaft erwiesen.

### Legenden zu den Abbildungen:

Abbildung 1:
   Physikalische Karte des Plasmids pLIP3+ (7884 bps).
Abbildung 2:
   Physikalische Karte des Plasmids pBBL7 (7552 bps).
Abbildung 3:
   Ligation/Amplifikation des Strukturgens *pil*D/*xcp*A (Beispiel 3).

Die folgenden Beispiele erläutern die Erfindung weiter:

### Beispiel 1

### Expression des Lipaseoperons lipAH aus Pseudomonas aeruginosa PAO1 in Pseudomonas putida BMTU650

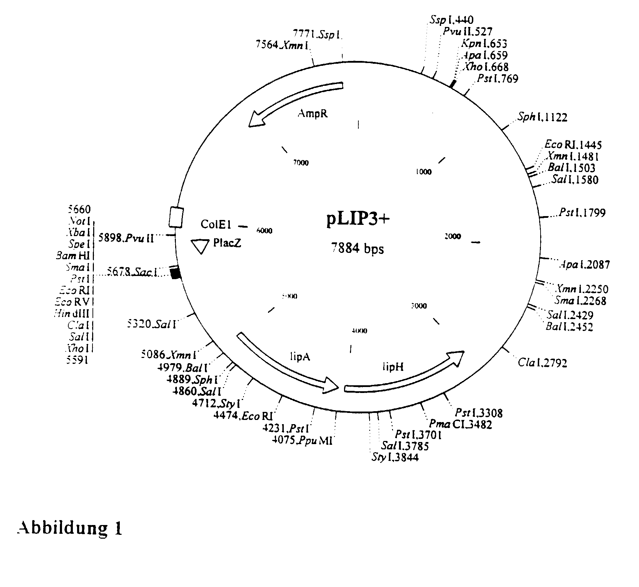

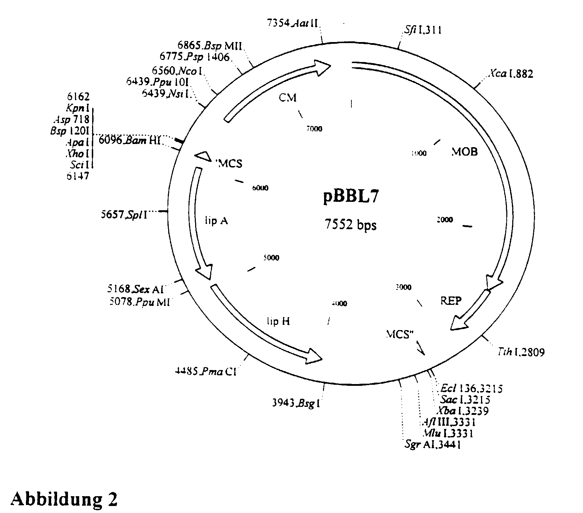

Um die Expression des Lipaseoperons in *P*. *putida*, z.B. in BMTU650 (DSM 12384), zu ermöglichen, wurde das Plasmid pBBL7 konstruiert. Dazu wurde das Lipaseoperon *lipA*/*lipH* aus dem Plasmid pLIP3+ (Abbildung 1) als 2.8 kb *Xmnl*-*SmaI* Fragment isoliert und in den mit *SmaI* linearisierten Vektor mit weitem Wirtsbereich pBBR1MCS (Kovac et al., Bio Techniques vol. 16 (1994), 800-802) ligiert. Nach Transformation von *E.coli* JM109 und der Analyse Chloramphenicol-resistenter Klone wurde das gesuchte Plasmid pBBL7 (Abbildung 2) identifiziert. Im Plasmid pBBL7 befindet sich das Lipaseoperon stromabwärts einer *E.coli lac*-Promotor-Sequenz.

Die Transformation von *P*. *putida* mit dem Plasmid pBBL7 erfolgte anschließend konjugativ durch sogenanntes "diparental mating" unter Zuhilfenahme von *E.coli* S17-1 (Simon et al., Meth. Enzymol. vol. 118 (1986), 640-659).

Die erhaltenen *P*. *putida*-Transkonjuganden wurden anschließend auf die Produktion extrazellulärer Lipaseaktivität nach Anzucht in LB-Flüssigmedium (Sambrook et al. (1989) Molecular Cloning: A laboratory manual: Cold Spring Harbour Press, New York) untersucht. Dazu wurden 50 ml NB-Medium mit Bakterien einer stationären Bakterienkultur mit 1x10⁸ Zellen beimpft und 18h bei 30°C und 220 Upm bebrütet. Die Lipaseaktivität im Kulturüberstand wurde mit p-Nitrophenylpalmitat als Substrat nach der bei Winkler & Stuckman beschriebenen Methode (J. Bacteriol. vol. 138 (1979), 663-670) ermittelt. Zusätzlich wurde die Bildung einer extrazellulären Lipase auf Tributyrin-Indikatoragarplatten (Kok et al. J. Gen. Microbiol. vol. *139* (1993), 2329-2343) verfolgt. Dazu wurden Einzelkolonien auf den Indikatoragar überführt; die Agarplatten 18h bei 30°C bebrütet und ausgewertet. Mit beiden Methoden konnte keine extrazelluläre Lipaseaktivität detektiert werden (Tabelle 1).

Ergebnis: Die extrazelluläre Lipaseaktivität des transformierten Bakteriums *P*. *putida* wurde mit zwei verschiedenen Methoden bestimmt: Weder mittels eines Plattentests mit dem Lipase-Substrat Tributyrin, noch durch die Bestimmung der extrazellulären Lipaseaktivität im Kulturüberstand mit dem Substrat p-Nitrophenylpalmität konnte extrazelluläre Lipaseaktivität nachgewiesen werden (Tabelle 1); d.h. *P. putida* ist ohne zusätzliche Sekretionsgene nicht in der Lage, heterologe Proteine in das umgebende Kulturmedium zu sekretieren.

### Beispiel 2

### Sekretion der Lipase aus P. aeruginosa PAO1 in P. putida BMTU650

Um *P. putida* BMTU650 (DSM 12384) den Export der Lipase aus *P. aeruginosa* PAO1 zu ermöglichen, wurden die für die Sekretion der Lipase verantwortlichen Gene aus einer *P. aeruginosa*-Genbank kloniert. Die Genbank wurde in dem Cosmid pLAFR3 (Staskawicz et al., J. Bacteriol. vol. 169 (1987), 5789-5794) angelegt. Dieses Cosmid besitzt einen RK-2 Replikationsursprung mit weitem Wirtsbereich, vermittelt Resistenz gegen Tetrazyklin und ist mobilisierbar, nicht aber selbst-übertragbar.

Zur Herstellung der Genbank wurden 50 µg chromosomaler DNA aus *P. aeruginosa* PAO1 mit *Sau*3Al partiell verdaut und in einem Saccharose-Dichtegradienten der Größe nach getrennt. DNA-Fragmente mit einer Größe von 12-25 kb wurden in das Cosmid pLAFR3 ligiert. Die Vorbereitung der Cosmid-DNA zur Ligation folgte der bei Ish-Horowicz (Nucleic Acids Res. vol. 9, (1981) 2989-2998) beschriebenen Methode. Die Verpackung der ligierten PAO1/pLAFR3-DNA in *E.coli* λ-Phagen-Köpfe erfolgte unter Zuhilfenahme eines kommerziell erhältlichen Verpackungsextrakt nach den Angaben des Herstellers (λ-DNA *in vitro* packaging module; Amersham). Die anschließende Transfektion der ligierten DNA in *E.coli* DH5α folgte bekannten Standardmethoden (Sambrook et al. (1989) Molecular Cloning: A laboratory manual. Cold Spring Harbour Press, New York).

Es wurden ca. 15.000 Tetrazyklin-resistente Transfektanten erhalten: die durchschnittliche Insertgröße betrug ca. 18-20 kb. Geht man von einer *P. aeruginosa*-Genomgröße von ca. 6000 kb (Holloway et al., Microbiology vol. 140 (1994), 2907-2929) und einer durchschnittlichen Cosmid-Insertgröße von 18 kb aus, so repräsentieren ca. 1530 unabhängige Klone 99% des gesamten Genoms (Clark und Carbon, Cell vol. 9 (1976), 91), d.h. das eine bestimmte DNA-Sequenz mit einer 99% Wahrscheinlichkeit in der Genbank enthalten ist.

Um die für die Sekretion der Lipase verantwortlichen Gene zu isolieren, wurden jeweils 960 Einzelklone vereinigt und die Cosmid-DNA in *P. putida* BMTU650/pBBL7 mobilisiert. Dazu wurde die "triparental mating"-Technik wie bei Friedman et al., Gene vol. 18 (1982), 289-296 beschrieben, unter Beteiligung des Helferbakteriums *E.coli* DH5α/pRK2013, angewandt. Einer der so erhaltenen Transkonjuganden wies auf Tributyrin-Indikatoragarplatten (Kok et al. (1993), s.o.) eine starke Hofbildung auf, was extrazelluläre Lipaseaktivität anzeigt. Das in diesem Cosmid enthaltene Plasmid wurde mit pLAXCP7 bezeichnet. Nach Anzucht in LB-Flüssigmedium produzierte dieser Klone mit ca. 1mg/ml extrazellulärer Lipase (Tabelle 1). Damit übersteigt die extrazelluläre Lipasemenge des Stamms *P. putida* BMTU650/pBBL7/pLAXCP7 die des *P. aeruginosa* PAO1um einen Faktor von ca. 30 (Stuer et al., J. Bacteriol. 168 (1986), 1070-1074).

Ein mit dem isolierten Cosmid pLAXCP7 retransformierter *P. putida* BMTU650 wies keine Hofbildung auf Tributyrin-Indikatoragarplatten und keine extrazelluläre Lipaseaktivität nach Anzucht in LB-Flüssigmedium (Sambrook et al., s.o.) auf. Dies zeigt, daß auf dem Cosmid pLAXCP7 kein weiteres lipotlytisches Enzym, sondern tatsächlich die für den Export der auf dem Plasmid pBBL7 kodierten Lipase aus *P. aeruginosa* PAO1 verantwortlichen Gene lokalisiert sind.

Ebenso wies *E.coli* JM 109 (Yanisch-Perron et al. Gene 33(1985). 103-119), mit den Plasmiden pBBL7 oder pLAXCP7 alleine oder mit beiden Plasmiden gleichzeitig transformiert, keine extrazelluläre Lipaseaktivität auf (Tabelle 1).

Mittels Restriktionsanalysen, DNA-DNA-Hybridisierung (Southern, J. Mol. Biol. vol. 98 (1975), 503) mit geeigneten, homologen Sonden sowie durch genetische Komplementation zweier *P. aeruginosa xcp*-Mutanten, konnte gezeigt werden, daß pLAXCP7 das gesamte bekannte *xcp*-Operon trägt.

**Tabelle 1**

| Extrazelluläre Lipaseaktivität von *P. putida* BMTU 650 und *E.coli* JM 109 sowie deren mit den Plasmiden pBBL7 und/oder pLAXCP7 transformierten Derivaten im Vergleich zu *P. aeruginosa* PAO1 | | |
|---|---|---|
| Bakterienstamm | Lipaseaktivität [µkat)/I] im Kulturmedium | Hofbildung auf Tributyrin-Indikatoragarplatten |
| *P. aeruginosa* PAO1 | 22.8 | + |
| *P. putida* BMTU650 | 0 | - |
| *P. putida* BMTU650/pBBL7 | 0 | - |
| *P. putida* BMTU650/pLAXCP7 | 0 | - |
| *P. putida* BMTU650/pBBL7/pLAXCP7 | 760 | +++ |
| *P. putida*/ BMTU650/pBBL7/pLAXCP7/pUSSpiL | 1140 | +++ |
| *E.coli* JM 109 | 0 | - |
| *E.coli* JM 109/pBBL7 | 0 | - |
| *E.coli* JM 109/pLAXCP7 | 0 | - |
| *E.coli* JM109/pBBL7/pLAXCP7 | 0 | - |

Somit konnte erstmalig ein extrazelluläres Enzym/Protein, das aus einem der Sicherheitsstufe 2 zugehörigen Mikroorganismus stammt, und dessen Expression in *E.coli* bei Co-Expression der homologen Sekretionsgene nicht zur Produktion eines aktiven zellulären Enzyms führt, in einem heterologen Wirtsorganismus - darüber hinaus wirtschaftlichen Maßstäben genügend - exprimiert und durch Co-Expression geeigneter Sekretionsgene spezifisch in das umgebende Kulturmedium sekretiert werden.

### Beispiel 3

### Steigerung der Sekretion von Lipase aus P. aeruginosa PAO1 in P. putida BMTU650

Um die Ausbeute an exponierter, enzymatisch aktiver Lipase aus *P. aeruginosa* PAO1 in *P. putida* BMTU650 (Beispiel 2) zu erhöhen, wurde die für die Assemblierung des Sekretionsapparats notwendige Präpilin-Peptidase PilD/XcpA (Bally et al., J. Bacteriol 173 (1991), 479-486) aus *P. aeruginos*a in einen Expressionsvektor mit weitem Wirtsbereich kloniert.

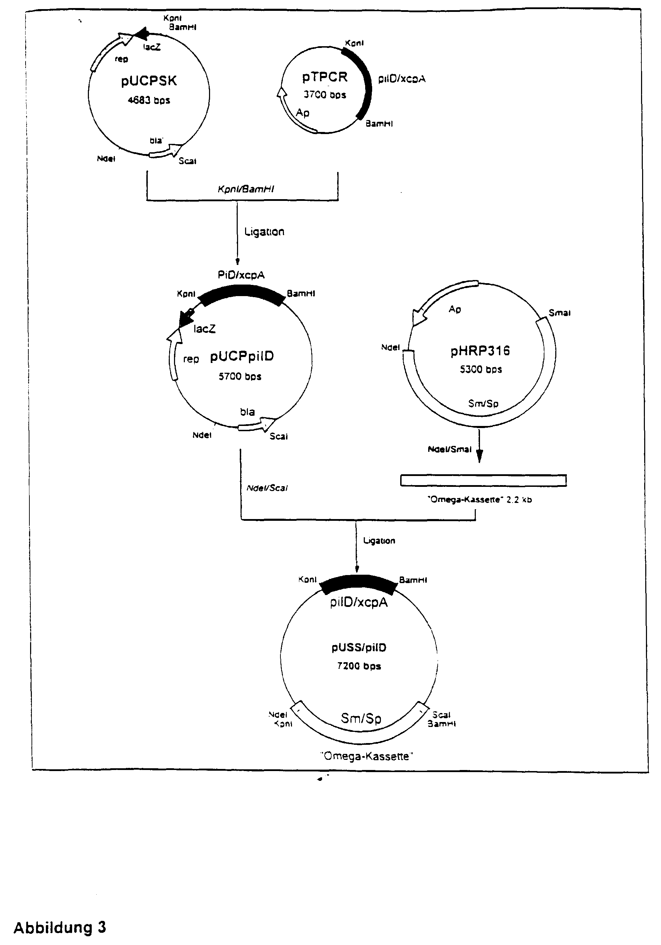

Hierzu wurde das Strukturgen *pil*D/*xp*A unter Verwendung synthetischer, homologer Primermoleküle und chromosomaler DNA aus *P. aeruginosa* PAO1 in einer Polymerase-Kettenreaktion (PCR) amplifiziert. Das erhaltene 1,1 kb große PCR-Fragment wurde zunächst in einen modifizierten pUC18-Sequenzierungsvektor (Marchuk et al., Nucl. Acids Res. 19 (1990)) kloniert Abbildung 3). Durch DNA-Sequenzierung wurde das erhaltene DNA-Fragment als das Strukturgen *pil*D/*xcp*A identifiziert.

Zur Expression der Präpilin-Peptidase im heterologen Wirt wurde das Strukturgen *pil*D/*xcp*A aus dem Sequenzierungsvektor durch Restriktion mit den Restriktionsendonukleasen *Bam*HI und *Kpn*I isoliert und in dem ebenso restringierten Plasmidvektor pUCPSK (Watson et al., Gene 172 (1996), 163-164) subkloniert (Abbildung 3). Dieser Vektor weist einen weiten Wirtsbereich auf, besitzt einen *lac*- und T7-Promotor und ist durch eine Ampicillin-Resistenz selektierbar. Durch die *Bam*HI/*Kpn*I-Klonierung steht das *pil*D/*xcp*A Gen in dem entstandenen Plasmid pUCPpilD unter transkriptioneller Kontrolle des *lac*-Promotors.

*P. putida* BMTU650 weist wie zahlreiche Pseudomonaden eine hohe intrinsische Resistenz gegen Derivate des Antibiotikums Penicillin auf, so daß beispielsweise Ampicillin zur Selektion von Plasmiden in diesen Stämmen nicht geeignet ist. Daher wurde das Ampicillin-Resistenzgen in dem Plasmid pUCPpiLD durch Insertion einer sog. Ω-Kassette aus dem Plasmid pHRP316 (Parales & Harwood. Gene 166, (1993), 23-30) durch Restriktion als 2.2 kb *Smal*/*Nde*I-Fragment isoliert und in das durch *Nde*I/*Sca*I-Restriktion unter Verlust von 692 bps linearisierte Plasmid pUCPpilD ligiert. Das so entstandene Plasmid pUSSpilD wurde durch Transformation in die *P. aeruginosa pil*D-Negativmutante PAK 2B18 (Nunn et al., J. Bacteriol., 172 (1990), 2911-2919) eingebracht, die ebenfalls das Plasmid pBBL7 trug. Eine starke Hofbildung auf Tributyrin-Indikatoragarplatten zeigte die vollständige Komplementation des *pil*D-negativen Phänotyps durch die von dem Plasmid pUSSpilD kodierte Präpilin-Peptidase PilD/XcpA.

Nach Transformation des Stammes *P. putida* BMTU650/pBBL7/pLAXCP7 mit dem Plasmid pUSSpilD, wies dieser Stamm eine um den Faktor 1,5 gesteigerte extrazelluläre Lipaseaktivität auf verglichen mit einem Kontrollstamm, der mit dem entsprechenden Leervektor pUSS ohne pilD-Strukturgen transformiert worden war (Tabelle 1).

## Patentansprüche

1. Verfahren zur Herstellung eines extrazellulären, mikrobiellen Proteins durch heterologe Expression in einem Kulturmedium, dadurch gekennzeichnet, daß man ein für das extrazelluläre Protein kodierendes Nukleinsäuremolekül und ein für einen Sekretionsapparat kodierendes Nukleinsäuremolekül aus dem entsprechenden Mikroorganismus isoliert, in einen Expressionsvektor kloniert und in einem heterologen, Gram-negativen Bakterium überexprimiert und anschließend das Protein aus dem Kulturmedium isoliert.

2. Verfahren nach Anspruch 1, dadurch gekennzeichnet, daß das für das extrazelluläre Protein kodierende Nukleinsäuremolekül und/oder das für den Sekretionsapparat kodierende Nukleinsäuremolekül in das Genom des Wirtsorganismus stabil integriert wird.

3. Verfahren nach Anspruch 1, dadurch gekennzeichnet, daß es sich bei dem Sekretionsapparat um einen Multiprotein-Komplex handelt.

4. Verfahren nach einem der Ansprüche 1, 2 oder 3, dadurch gekennzeichnet, daß der Sekretionsapparat den Transport des extrazellulären Proteins über die äußere Membran Gramnegativer Bakterien ermöglicht.

5. Verfahren nach einem der Ansprüche 1 bis 4, dadurch gekennzeichnet, daß es sich bei den Sekretionsapparat um ein Typ II-Sekretionssystem handelt.

6. Verfahren nach einem der Ansprüche 1 bis 5, dadurch gekennzeichnet, daß es sich bei den für den Sekretionsapparat kodierenden Nukleinsäuremolekülen um die *xcp*-Gene aus *P. aeruginosa* handelt.

7. Verfahren nach Anspruch 6, dadurch gekennzeichnet, daß zusätzlich zu den für den Sekretionsapparat kodierenden *xcp*-Genen ein für die Prozessierung der diesen Sekretionsapparat bildenden Proteine zuständiges Gen exprimiert wird.

8. Verfahren nach einem der Ansprüche 1 bis 7, dadurch gekennzeichnet, daß das überexprimierte Protein in das Kulturmedium sekretiert wird.

9. Verfahren nach einem der Ansprüche 1 bis 8, dadurch gekennzeichnet, daß der Wirtsorganismus mehrere Nukleinsäuremoleküle, die für unterschiedliche extrazelluläre Proteine kodieren, enthält.

10. Verfahren nach einem der Ansprüche 1 bis 9, dadurch gekennzeichnet, daß das für ein extrazelluläres Protein kodierende Nukleinsäuremolekül aus zwei oder mehreren funktionellen Einheiten besteht und diese Einheiten gegebenenfalls in zwei oder mehrere Expressionsvektoren kloniert und unabhängig voneinander überexprimiert werden.

11. Verfahren nach einem der Ansprüche 1 bis 10, dadurch gekennzeichnet, daß die Expression des für das extrazelluläre Protein kodierenden Nukleinsäuremoleküls unter der Kontrolle seines natürlichen Promotors oder eines konstitutiven oder induzierbaren Fremdpromotors steht.

12. Verfahren nach einem der Ansprüche 1 bis 11, dadurch gekennzeichnet, daß das für den Sekretionsapparat kodierende Nukleinsäuremolekül aus einer oder mehreren funktionellen Einheiten besteht, und diese Einheit(en) auf einem oder gegebenenfalls mehreren Expressionsvektoren kloniert oder in das Genom integriert vorliegt (vorliegen) und unabhängig voneinander überexprimiert werden.

13. Verfahren nach einem der Ansprüche 1 bis 12, dadurch gekennzeichnet, daß das für den Sekretionsapparat kodierende Nukleinsäuremolekül unter der Kontrolle seines natürlichen Promotors oder eines konstitutiven oder induzierbaren Fremdpromotors steht.

14. Verfahren nach Anspruch 13, dadurch gekennzeichnet, daß die funktionellen Einheiten des für ein extrazelluläres Protein kodierenden Nukleinsäuremoleküls die Gene *lip*A und/oder *lipH* sind, wobei mindestens ein Gen gegebenenfalls mit einer für eine Signalsequenz eines Proteins kodierenden Nukleinsäureregion versehen ist.

15. Verfahren nach Anspruch 14, dadurch gekennzeichnet, daß das Gen *lipA* am 5'-Ende mit der genannten Nukleinsäureregion verbunden ist.

16. Verfahren nach Anspruch 14 oder 15, dadurch gekennzeichnet, daß die Signalsequenz der aus *E.coli* stammenden Gene *ompA*, *omp*T oder *phoA* oder des aus *Erwinia chrysanthemi* stammenden Gens pelB verwendet wird.

17. Verfahren nach einem der Ansprüche 1 bis 13, dadurch gekennzeichnet, daß das extrazelluläre Protein esterolytische Aktivität aufweist.

18. Verfahren nach einem der Ansprüche 1 bis 17, dadurch gekennzeichnet, daß das extrazelluläre Protein Lipase- und/oder Elastase- und/oder Protease-Aktivität aufweist.

19. Verfahren nach einem der Ansprüche 1 bis 18, dadurch gekennzeichnet, daß es sich bei dem heterologen Wirtsorganismus um ein Gram-negatives Bakterium der Gattung *Pseudomonas* handelt.

20. Verfahren nach Anspruch 19, dadurch gekennzeichnet, daß ein *Psuedomonas*-Stamm der Art *putida* oder *fluorescens* verwendet wird.
